# EUROPÄISCHE PATENTSCHRIFT

(11) **EP 3 498 217 B1**
(45) Veröffentlichungstag und Bekanntmachung des Hinweises auf die Patenterteilung: **25.06.2025**
(21) Anmeldenummer: 18210933.0
(22) Anmeldetag: 07.12.2018
(51) Int. Cl.: A61B 1/00, A61B 90/50, A61B 90/57, F16C 11/04

(54) **MEDIZINTECHNISCHE EINRICHTUNG**
MEDICAL TECHNOLOGY EQUIPMENT
DISPOSITIF MÉDICOTECHNIQUE

(30) Priorität: 13.12.2017 DE 102017129692
(43) Veröffentlichungstag der Anmeldung: 19.06.2019
(73) Patentinhaber: Karl Storz SE & Co. KG, 78532 Tuttlingen (DE)
(72) Erfinder: Egle, Michael, 78532 Tuttlingen (DE)

(56) Entgegenhaltungen:
- EP-A2- 2 223 659
- WO-A1-03/017859
- DE-A1- 102013 002 498
- DE-C- 906 871
- GB-A- 1 176 366
- US-A- 3 832 022
- US-A- 4 373 739
- US-A- 4 491 436
- US-A1- 2004 086 325
- US-A1- 2013 144 395
- US-A1- 2017 215 979
- US-B2- 9 131 987

## Beschreibung

Die Erfindung betrifft eine medizintechnische Einrichtung mit einer Gelenkanordnung, die für eine Schwenkbewegung eines ersten Gelenkteils gegenüber einem zweiten Gelenkteil ausgebildet ist, mit einem ersten Gelenkteil, das zwei beabstandet zueinander angeordnete Gelenkschenkel sowie einen zwischen den Gelenkschenkeln entlang einer Längsachse erstreckten Gelenkbolzen umfasst, der eine Schwenkachse der Gelenkanordnung bestimmt, sowie mit einem zweiten Gelenkteil, das bereichsweise zwischen den beiden Gelenkschenkeln aufgenommen ist und das von einer Gelenkausnehmung durchsetzt ist, wobei der Gelenkbolzen schwenkbeweglich in der Gelenkausnehmung aufgenommen ist.

Eine derartige Gelenkanordnung findet beispielsweise bei einem Halter für ein medizinisches Instrument Verwendung, wobei vorgesehen sein kann, das medizinische Instrument mit einer Klemmeinrichtung zu fixieren, wobei die Klemmeinrichtung eine Gelenkanordnung umfasst.

In US 2013/0144395 A1 ist eine als distale Handgelenkanordnung bezeichnetes Gelenkanordnung beschrieben. Ein zweiter Handgelenksrahmen umfasst einen distalen Gabelkopf mit zwei Zinken. Die Enden eines Drehzapfens sind in Durchgangslöchern in den Zinken aufgenommen. Zwischen den Zinken durchgreift der Drehzapfen ein Durchgangsloch in einem ersten Maulteil und an dessen Enden angrenzend Durchgangslöcher in einem proximal gabelförmigen zweiten Maulteil.

In US 4,491,436 ist eine Drehzapfenanordnung beschrieben. Die Drehzapfenanordnung umfasst eine Bohrung in einem Zwischenbauteil, einen Drehzapfen und ein reibungsminderndes Element das einen Innenring und einen Außenring enthält. Der Außenring ist in die Bohrung in dem Zwischenbauteil eingepresst und durch ein Paar Sprengringe in Position gehalten.

Die Aufgabe der Erfindung besteht darin, eine Gelenkanordnung bereitzustellen, die auch bei einer zeitweiligen Einleitung von hohen Kräften eine geringe Reibung für eine Schwenkbewegung der beiden Gelenkteile zueinander gewährleistet.

Diese Aufgabe wird für eine Gelenkanordnung der eingangs genannten Art mit den Merkmalen des Anspruchs 1 gelöst. Hierbei ist vorgesehen, dass der Gelenkbolzen und die Gelenkausnehmumg einen zentralen Kontaktbereich mit einem Berührkontakt zwischen Gelenkbolzen und Gelenkausnehmumg und beidseitig an den Kontaktbereich anschließende, bis zu den Gelenkschenkeln erstreckte Freistellungsbereiche ohne Berührkontakt zwischen Gelenkbolzen und Gelenkausnehmumg aufweist, um eine ausschließlich zentrale Kraftübertragung zwischen dem Gelenkbolzen und der Gelenkausnehmung zu gewährleisten.

Durch die ausschließlich zentrale Kraftübertragung wird erreicht, dass der Gelenkbolzen abseits des Kontaktbereichs in den beiden Freistellungsbereichen bei Einleitung von Kräften auf die beiden Gelenkteile eine Ausweichbewegung vollziehen kann und dabei elastisch deformiert werden kann. Dadurch können unerwünschten Reibungseffekte zwischen dem Gelenkbolzen und der Gelenkausnehmung vermieden werden, die langfristigen zu einer Schädigung der Gelenkanordnung führen könnten. Bei einer Krafteinleitung und/oder einer Drehmomenteinleitung auf die beiden Gelenkteile wird der Gelenkbolzen abseits des Kontaktbereichs im jeweiligen Freistellungsbereich S-förmig deformiert. Sofern eine Krafteinleitung auf die beiden Gelenkteile ausschließlich in einer Richtung quer zur Längsachse erfolgt, wird der Gelenkbolzen spiegelsymmetrisch zu einer Spiegelebene, die normal zur Längsachse ausgerichtet ist, S-förmig deformiert. Erfolgt hingegen eine Drehmomenteinleitung auf die beiden Gelenkteile, wobei das Drehmoment um eine quer zur Längsachse ausgerichtete Drehmomentachse, so findet eine S-förmige Deformation des Gelenkbolzens in den jeweiligen Freistellungsbereiche Achsen symmetrisch zur Drehmomentachse statt.

Vorteilhafte Weiterbildungen der Erfindung sind Gegenstand der Unteransprüche.

Zweckmäßig ist es, wenn die Gelenkausnehmung als Stufenbohrung mit einer zentral angeordneten Führungsbohrung ausgebildet ist, die einen geringeren Querschnitt als beidseitig daran angrenzende Freistellungsbohrungen aufweist. Hierdurch wird eine kostengünstige Herstellung der Führungsbohrung ermöglicht, da nur die Freistellungsbohrungen einen größeren Querschnitt aufweisen müssen, während die Führungsbohrung einen geringeren Querschnitt aufweist, dessen Profilierung derart auf eine Profilierung des Gelenkbolzens angepasst ist, dass der Gelenkbolzen mit der Führungsbohrung ein Gleitlager ausbilden kann. Bevorzugt ist vorgesehen, dass die Führungsbohrung und die Freistellungsbohrungen jeweils kreiszylindrisch ausgebildet sind und insbesondere koaxial zueinander angeordnet sind. Ergänzend kann vorgesehen sein, dass die Querschnitte der beiden Freistellungsbohrungen identisch sind.

Vorzugsweise ist vorgesehen, dass der Gelenkbolzen zwischen den Gelenkschenkeln mit einer konstanten Profilierung ausgebildet ist. Vorzugsweise weist der Gelenkbolzen eine kreisrunde Profilierung auf und ist somit als Kreiszylinder ausgeführt, wodurch sich eine kostengünstige Herstellungsweise für den Gelenkbolzen verwirklichen lässt.

Bei einer alternativen Weiterbildung der Erfindung ist vorgesehen, dass der Gelenkbolzen mit einem zentral angeordneten Führungsabschnitt ausgebildet ist, der einen größeren Querschnitt als beidseitig daran angrenzende Freistellungsabschnitte aufweist. Hierdurch lässt sich eine besonders vorteilhafte Elastizität für den Gelenkbolzen verwirklichen, da der zentral angeordneten Führungsabschnitt aufgrund seines größeren Querschnitts im Rahmen der für die Gelenkanordnung vorgesehenen Maximalkräfte formstabil ausgebildet werden kann, während die daran angrenzenden Freistellungsabschnitte aufgrund des geringeren Querschnitts bereits bei Kräften deutlich unterhalb der für die Gelenkanordnung vorgesehenen Maximalkräfte elastisch deformiert werden können. Vorzugsweise sind sowohl der Führungsabschnitt als auch die daran angrenzenden Freistellungsabschnitte jeweils kreiszylindrisch ausgebildet und koaxial zueinander angeordnet.

In weiterer Ausgestaltung der Erfindung ist vorgesehen, dass die die Gelenkausnehmung als Bohrung mit einer konstanten Profilierung ausgebildet ist. Hierdurch kann die Gelenkausnehmung durch einen einzigen Fertigungsvorgang, insbesondere einen Bohrvorgang, hergestellt werden.

Vorteilhaft ist es, wenn der Gelenkbolzen mit einem zentral angeordneten Führungsabschnitt ausgebildet ist, der einen größeren Querschnitt als beidseitig daran angrenzende Freistellungsabschnitte aufweist und dass die Gelenkausnehmung als Stufenbohrung mit einer zentral angeordneten Führungsbohrung ausgebildet ist, die einen geringeren Querschnitt als beidseitig daran angrenzende Freistellungsbohrungen aufweist. Durch die Anpassung der Querschnitte der Freistellungabschnitte auf die Querschnitte der Freistellungsbohrungen kann eine vorteilhafte Einstellung der gewünschten Elastizität für die Gelenkanordnung erzielt werden.

Bevorzugt ist vorgesehen, dass einander gegenüberliegende Oberflächen der Gelenkschenkel mit benachbarten Oberflächen des zweiten Gelenkteils eine spielbehaftete Gleitführung bilden und dass der Gelenkbolzen und die Gelenkausnehmung derart aufeinander abgestimmt sind, dass bei einer Verkippung des zweiten Gelenkteils gegenüber dem ersten Gelenkteil um eine quer zur Längsachse ausgerichtete Verkippungsachse eine ausschließlich elastische Deformation des Gelenkbolzens vorliegt. Die Gleitführung zwischen den beiden Gelenkteilen dient insbesondere zur Aufnahme von Kräften bzw. Kraftkomponenten, die parallel zur Längsachse des Gelenkbolzens ausgerichtet sind und nicht durch den Gelenkbolzen und die korrespondierende Gelenkausnehmung abgestützt werden. Darüber hinaus dient die Gleitführung zwischen den beiden Gelenkteilen für eine Begrenzung einer Verkippung zwischen dem ersten Gelenkteil und dem zweiten Gelenkteil um eine quer zur Längsachse ausgerichtete Verkippungsachse. Aufgrund der Elastizität des Gelenkbolzens können im Vergleich zu bekannten Gelenkanordnungen, die mit starren Gelenkbolzen verwirklicht sind, größere Verkippungswinkel für die beiden Gelenkteile zueinander akzeptiert werden. Hierdurch kann beispielsweise eine Klemmhalterung, die mit einer erfindungsgemäßen Gelenkanordnung ausgerüstet ist, sich in günstiger Weise an unterschiedliche Außengeometrien von zu klemmenden Gegenständen anpassen, ohne dass hierdurch eine unerwünschte Schwergängigkeit der Gelenkanordnung in Kauf genommen werden müsste.

Zweckmäßig ist es, wenn eine Erstreckung des zentralen Kontaktbereichs in Richtung der Längsachse weniger als 30 Prozent, vorzugsweise weniger als 25 Prozent, einer Erstreckung eines der Freistellungsbereiche in Richtung der Längsachse beträgt.

Bei einer vorteilhaften Weiterbildung der Erfindung ist vorgesehen, dass eine Querschnittsfläche der Führungsbohrung weniger als 50 Prozent, vorzugsweise weniger als 40 Prozent, insbesondere weniger als 30 Prozent, einer Querschnittsfläche der Freistellungsbohrung beträgt.

In weiterer Ausgestaltung der Erfindung ist vorgesehen, dass eine Querschnittsfläche des Freistellungsabschnitts weniger als 50 Prozent, vorzugsweise weniger als 40 Prozent, insbesondere weniger als 30 Prozent, einer Querschnittsfläche des Führungsabschnitts beträgt.

Vorteilhafte Ausführungsformen der Erfindung sind in der Zeichnung dargestellt. Hierbei zeigt:
- Figur 1: eine schematische Darstellung einer medizintechnischen Einrichtung, die eine Gelenkanordnung als Bestandteil einer Klemmeinrichtung umfasst,
- Figur 2: eine schematische Detaildarstellung der Klemmeinrichtung gemäß der Figur 1,
- Figur 3: eine schematische Draufsicht auf die Klemmeinrichtung gemäß der Figur 2,
- Figur 4: eine Detaildarstellung der Klemmeinrichtung gemäß den Figuren 2 und 3 mit einer ersten Ausführungsform einer Gelenkanordnung, bei der der Gelenkbolzen mit einem zentral angeordneten Führungsabschnitt ausgebildet ist, der einen größeren Querschnitt als beidseitig daran angrenzende Freistellungsabschnitte aufweist,
- Figur 5: eine Detaildarstellung der Klemmeinrichtung gemäß den Figuren 2 und 3 mit einer zweiten Ausführungsform einer Gelenkanordnung, bei der die Gelenkausnehmung als Stufenbohrung ausgebildet ist und der Gelenkbolzen eine konstante Profilierung aufweist,
- Figur 6: eine schematisierte Darstellung eines Gelenkbolzens bei einer ausschließlichen Einleitung von Radialkräften, und
- Figur 7: eine schematisierte Darstellung eines Gelenkbolzens bei einer ausschließlichen Einleitung eines Drehmoments um eine quer zu einer Längsachse des Gelenkbolzens ausgerichteten Drehmomentachse.

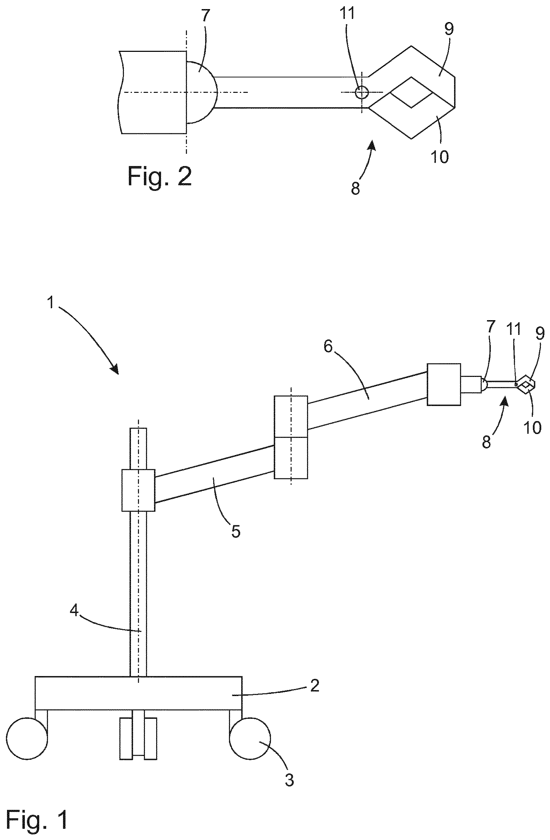

Eine in der Figur 1 dargestellte medizintechnische Einrichtung 1 ist rein exemplarisch als fahrbarer Tragarm für ein nicht näher dargestelltes medizinisches Instrument, beispielsweise ein endoskopisches Kamerasystemen, ausgebildet.

Die medizintechnische Einrichtung 1 umfasst ein Grundgestell 2, das mit Rollen 3 ausgestattet ist und das eine Säule 4 aufweist, die sich im Wesentlichen in vertikaler Richtung nach oben erstreckt. An der Säule 4 ist ein erster Tragarmabschnitt 5 höhenverstellbar und um eine vertikale Achse schwenkbar angeordnet, der an einem der Säule 4 abgewandten Endbereich mit einem zweiter Tragarmabschnitt 6 gekoppelt ist, der seinerseits um eine nicht dargestellte vertikale Achse schwenkbeweglich am ersten Tragarmabschnitt 5 gelagert ist.

An einem dem ersten Tragarmabschnitt 5 abgewandten Endbereich des zweiten Tragarmabschnitts 6 ist rein exemplarisch unter Zwischenschaltung eines Kugelgelenks 7 eine Greifanordnung 8 ausgebildet, die eine feststehende Greiferklaue 9 sowie eine bewegliche Greiferklaue 10 umfasst. Dabei bilden die feststehende Greiferklaue 9 und die bewegliche Greiferklaue 10 eine Gelenkanordnung 11, die die schwenkbewegliche Relativbewegung der beweglichen Greiferklaue 10 gegenüber der feststehenden Greiferklaue 9 ermöglicht.

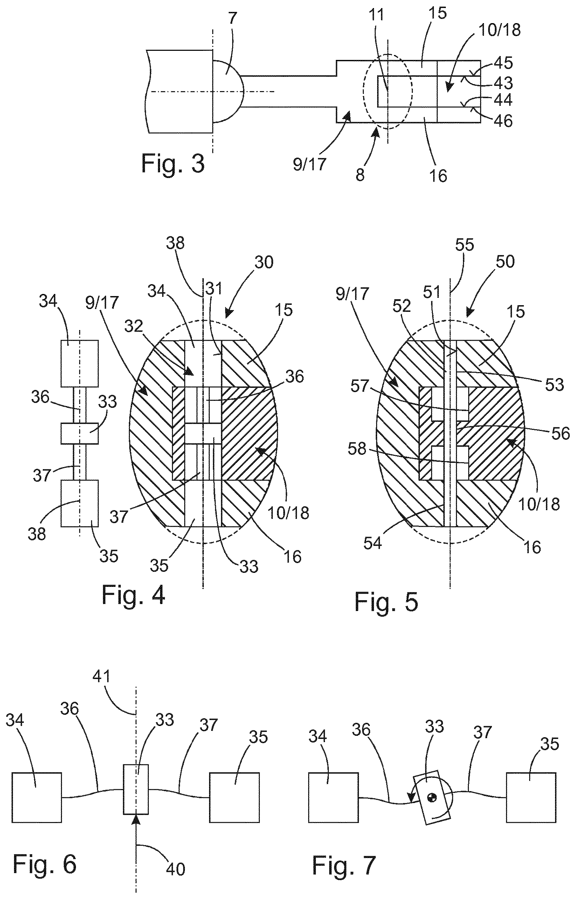

Wie aus der schematischen Darstellung der Figur 3 entnommen werden kann, ist die feststehende Greiferklaue 9 an einem dem Kugelgelenk 7 abgewandten Endbereich gabelförmig ausgebildet und umfasst zwei beabstandet zueinander angeordnete Gelenkschenkel 15, 16, zwischen denen rein exemplarisch die bewegliche Greiferklaue 10 aufgenommen ist. Somit bildet die feststehende Greiferklaue 9 mit ihren Gelenkschenkeln 15, 16 ein erstes Gelenkteil 17, während die bewegliche Greiferklaue 10 ein zweites Gelenkteil 18 bildet. Die beiden Gelenkteile 17, 18 sind mittels eines Gelenkbolzens schwenkbeweglich miteinander verbunden, wobei in den Figuren 4 und 5 unterschiedliche Ausführungsformen von Gelenkanordnungen dargestellt sind.

Rein exemplarisch sind in der Figur 3 die einander zugewandten Oberflächen 43, 44 der beiden Gelenkschenkel 15, 16 und die einander entgegengesetzten Oberflächen 45, 46 des zweiten Gelenkteils 18 derart angeordnet, dass kein Lagerspiel für das Gleitlager erkennbar ist, das durch diese Oberflächen 43 bis 46 für die beiden Gelenkteile 17, 18 gebildet wird. In der Praxis werden die jeweils gegenüberliegenden Oberflächen 43 und 45 bzw. 44 und 46 jeweils einen geringfügigen Abstand zueinander aufweisen, um eine Leichtgängigkeit für die Gelenkanordnung 11 nicht in Frage zu stellen. Dementsprechend kann das zweite Gelenkteil 18 bei Einleitung eines Drehmoments, das normal zur Darstellungsebene der Figur 3 ausgerichtet ist, eine Verkippung des zweiten Gelenkteils 18 gegenüber dem ersten Gelenkteil 17 hervorrufen, wie dies symbolisch in der Figur 7 dargestellt ist.

Bei der in Figur 4 dargestellten ersten Ausführungsform einer Gelenkanordnung 30 sind die beiden Gelenkschenkel 15 und 16 des ersten Gelenkteils 17 wie auch das zweite Gelenkteil 18 von einer rein exemplarisch kreiszylindrisch ausgebildeten Durchgangsbohrung 31 durchsetzt, die im Hinblick auf das zweite Gelenkteil 18 auch als Gelenkausnehmung bezeichnet werden kann. In der Durchgangsbohrung 31 ist ein zur Verdeutlichung seiner Geometrie zusätzlich zur Schnittdarstellung der Figur 4 als Einzelteil dargestellter Gelenkbolzen 32 vorgesehen, der beispielsweise reibschlüssig in den beiden Gelenkschenkeln 15 und 16 aufgenommen ist und der mit dem zweiter Gelenkteil 18 ein Schwenklager bildet. Hierzu umfasst der Gelenkbolzen 32 einen zentral angeordneten Führungsabschnitt 33, der an ein, vorzugsweise kreisrundes, Profil der Durchgangsbohrung 31 derart angepasst ist, dass eine gleitbeweglich Relativbewegung zwischen dem Führungsabschnitt 33 und dem mit der Durchgangsbohrung 31 versehenen zweiter Gelenkteil 18 ermöglicht wird. Jeweils endseitig ist der Gelenkbolzen 32 mit Halteabschnitten 34, 35 versehen, die kraftschlüssig in den Gelenkschenkeln 15, 16 festgelegt sind und damit eine drehfeste Verbindung zwischen dem Gelenkbolzen 32 und dem ersten Gelenkteils 17 gewährleisten.

Beidseitig des Führungsabschnitts 33 erstrecken sich entlang einer als Schwenkachse für das zweite Gelenkteil 18 dienenden Längsachse 38 jeweils Freistellungsabschnitte 36, 37 bis zu den Halteabschnitten 34 bzw. 35. Dabei weisen die Freistellungsabschnitte 36 und 37 einen erheblich kleineren Querschnitt, insbesondere einen erheblich kleineren Durchmesser, als der Führungsabschnitt 33 auf. Durch diese geometrische Ausgestaltung des Gelenkbolzens 32 ist gewährleistet, dass eine Kraftübertragung zwischen dem zweiter Gelenkteil 18 und dem am ersten Gelenkteil 17 festgelegten Gelenkbolzen 32 ausschließlich im Bereich des Führungsabschnitts 33 vorliegt und dass die Freistellungsabschnitte 36 und 37 aufgrund ihrer verglichen mit dem Führungsabschnitt 33 erheblich geringeren Querschnitte eine vorteilhafte Elastizität für die Gelenkanordnung 33 ermöglichen.

Bei der in Figur 5 dargestellten zweiten Ausführungsform einer Gelenkanordnung 50, bei der der Gelenkbolzen 52 kraftschlüssig in Führungsabschnitten 53, 54 der Gelenkschenkel 15, 16 aufgenommen ist und entlang einer Längsachse 55 eine konstante Profilierung aufweist, ist die als Durchgangsbohrung 51 ausgebildete Gelenkausnehmung als Stufenbohrung verwirklicht. Die Durchgangsbohrung 51 umfasst hierbei eine zentral angeordnete Führungsbohrung 56, deren Querschnitt auf den Querschnitt des Gelenkbolzens 52 angepasst ist, um ein schwenkbewegliches Gleitlager zu bilden. Abseits der Führungsbohrung 56 erstrecken sich beidseitig angrenzende Freistellungsbohrungen 57, 58, die die Freistellungsbereiche ohne Berührkontakt zwischen den Gelenkbolzen 52 und der als Gelenkausnehmung dienenden Durchgangsbohrung 51 gewährleisten.

Aus der rein schematischen Darstellung der Figuren 6 und 7, die sich auf den Gelenkbolzen 32 gemäß der erster Ausführungsform der Gelenkanordnung 30 beziehen, in ähnlicher Weise jedoch auch für die Gelenkanordnung 50 gelten, geht hervor, dass bei einer Einleitung einer rein radialen Kraft 40, die über das zweiter Gelenkteil 18 in den Führungsabschnitts 33 eingehängt wird, eine S-förmige Deformation der beiden Freistellungsabschnitte 36, 37 spiegelsymmetrisch zu einer Spiegelebene 41 erfolgt.

Bei einer Einleitung eines Drehmoments 42 um eine normal zur Darstellungsebene der Figur 7 ausgerichtete Drehmomentachse auf den Führungsabschnitts 33 ergibt sich hingegen eine Verkippung des Führungsabschnitts 33 gegenüber den Halteabschnitten 34 und 35, bei der die Freistellungsabschnitte 36 und 37 ebenfalls jeweils S-förmig deformiert werden, wobei hier die geometrische Beziehung zwischen den beiden Freistellungsabschnitte 36 und 37 eine Spiegelung um die Drehmomentachse ist.

## Patentansprüche

1. Medizintechnische Einrichtung mit einer Gelenkanordnung (11; 30; 50), die für eine Schwenkbewegung eines ersten Gelenkteils (17) gegenüber einem zweiten Gelenkteil (18) ausgebildet ist, mit einem ersten Gelenkteil (17), das zwei beabstandet zueinander angeordnete Gelenkschenkel (15, 16) sowie einen zwischen den Gelenkschenkeln (15, 16) entlang einer Längsachse (38; 55) erstreckten Gelenkbolzen (32; 52) umfasst, der eine Schwenkachse (38; 55) der Gelenkanordnung (11; 30; 50) bestimmt, sowie mit einem zweiten Gelenkteil (18), das bereichsweise zwischen den beiden Gelenkschenkeln (15 16) aufgenommen ist und das von einer Gelenkausnehmung (31; 51) durchsetzt ist, wobei der Gelenkbolzen (32; 52) schwenkbeweglich in der Gelenkausnehmung (31; 51) aufgenommen ist, wobei der Gelenkbolzen (32; 52) und die Gelenkausnehmumg (31; 51) einen zentralen Kontaktbereich (33; 56) mit einem Berührkontakt zwischen Gelenkbolzen (32; 52) und Gelenkausnehmumg (31; 51) und beidseitig an den Kontaktbereich (33; 56) anschließende, bis zu den Gelenkschenkeln (15, 16) erstreckte Freistellungsbereiche (36, 37; 57, 58) zumindest teilweise innerhalb der Gelenkausnehmung (31; 51) und ohne Berührkontakt zwischen Gelenkbolzen (32; 52) und Gelenkausnehmumg (31; 51) aufweist, um eine ausschließlich zentrale Kraftübertragung zwischen dem Gelenkbolzen (32; 52) und der Gelenkausnehmung (31; 51) zu gewährleisten.

2. Medizintechnische Einrichtung nach Anspruch 1, **dadurch gekennzeichnet, dass** die Gelenkausnehmung (51) als Stufenbohrung mit einer zentral angeordneten Führungsbohrung (56) ausgebildet ist, die einen geringeren Querschnitt als beidseitig daran angrenzende Freistellungsbohrungen (57, 58) aufweist.

3. Medizintechnische Einrichtung nach Anspruch 2, **dadurch gekennzeichnet, dass** der Gelenkbolzen (52) zwischen den Gelenkschenkeln (15, 16) mit einer konstanten Profilierung ausgebildet ist.

4. Medizintechnische Einrichtung nach Anspruch 1, **dadurch gekennzeichnet, dass** der Gelenkbolzen (32) mit einem zentral angeordneten Führungsabschnitt (33) ausgebildet ist, der einen größeren Querschnitt als beidseitig daran angrenzende Freistellungsabschnitte (36, 37) aufweist.

5. Medizintechnische Einrichtung nach Anspruch 4, **dadurch gekennzeichnet, dass** die die Gelenkausnehmung (31) als Bohrung mit einer konstanten Profilierung ausgebildet ist.

6. Medizintechnische Einrichtung nach Anspruch 1, **dadurch gekennzeichnet, dass** der Gelenkbolzen mit einem zentral angeordneten Führungsabschnitt ausgebildet ist, der einen größeren Querschnitt als beidseitig daran angrenzende Freistellungsabschnitte aufweist und dass die Gelenkausnehmung als Stufenbohrung mit einer zentral angeordneten Führungsbohrung ausgebildet ist, die einen geringeren Querschnitt als beidseitig daran angrenzende Freistellungsbohrungen aufweist.

7. Medizintechnische Einrichtung nach Anspruch 1, **dadurch gekennzeichnet, dass** einander gegenüberliegende Oberflächen (43, 44) der Gelenkschenkel (15, 16) mit benachbarten Oberflächen (45, 46) des zweiten Gelenkteils (18) eine spielbehaftete Gleitführung bilden und dass der Gelenkbolzen (32; 52) und die Gelenkausnehmung (31; 51) derart aufeinander abgestimmt sind, dass bei einer Verkippung des zweiten Gelenkteils (18) gegenüber dem ersten Gelenkteil (17) um eine quer zur Längsachse (38; 55) ausgerichtete Verkippungsachse eine ausschließlich elastische Deformation des Gelenkbolzens (32; 52) vorliegt.

8. Medizintechnische Einrichtung nach einem der vorhergehenden Ansprüche, **dadurch gekennzeichnet, dass** eine Erstreckung des zentralen Kontaktbereichs (33; 56) in Richtung der Längsachse (38; 55) weniger als 30 Prozent, vorzugsweise weniger als 25 Prozent, einer Erstreckung eines der Freistellungsbereiche (36, 37; 57, 58) in Richtung der Längsachse (38; 55) beträgt.

9. Medizintechnische Einrichtung nach Anspruch 2 oder 3, **dadurch gekennzeichnet, dass** eine Querschnittsfläche der Führungsbohrung (56) weniger als 50 Prozent, vorzugsweise weniger als 40 Prozent, insbesondere weniger als 30 Prozent, einer Querschnittsfläche der Freistellungsbohrung (57, 58) beträgt.

10. Medizintechnische Einrichtung nach Anspruch 4 oder 5, **dadurch gekennzeichnet, dass** eine Querschnittsfläche des Freistellungsabschnitts (36, 37) weniger als 50 Prozent, vorzugsweise weniger als 40 Prozent, insbesondere weniger als 30 Prozent, einer Querschnittsfläche des Führungsabschnitts (33) beträgt.

## Claims

1. A medical device having a joint arrangement (11; 30; 50) which is designed for a swivelling movement of a first joint part (17) relative to a second joint part (18), having a first joint part (17), which comprises two joint limbs (15, 16) arranged spaced apart from one another, and a joint pin (32; 52), which extends between the joint limbs (15, 16) along a longitudinal axis (38; 55) and which determines a pivot axis (38; 55) of the joint arrangement (11; 30; 50), and having a second joint part (18) which is received in regions between the two joint limbs (15, 16) and which is penetrated by a joint recess (31; 51), wherein the joint pin (32; 52) is received in a pivotally movable manner in the joint recess (31; 51), wherein the joint pin (32; 52) and the joint recess (31; 51) have a central contact region (33; 56) with a touching contact between joint pin (32; 52) and the joint recess (31; 51) and clearance regions (36, 37; 57, 58) which adjoin on both sides of the contact region (33; 56) and extend up to the joint limbs (15, 16), at least partially within the joint recess (31; 51) and without touching contact between joint pin (32; 52) and joint recess (31; 51), in order to ensure an exclusively central force transmission between the joint pin (32; 52) and the joint recess (31; 51).

2. The medical device according to claim 1, **characterised in that** the joint recess (51) is designed as a stepped bore with a centrally arranged guide bore (56) which has a smaller cross-section than the clearance bores (57, 58) adjoining it on both sides.

3. The medical device according to claim 2, **characterised in that** the joint pin (52) between the joint limbs (15, 16) is formed with a constant profiling.

4. The medical device according to claim 1, **characterised in that** the joint pin (32) is formed with a centrally arranged guide section (33) which has a larger cross-section than the clearance sections (36, 37) adjoining it on both sides.

5. The medical device according to claim 4, **characterised in that** the joint recess (31) is designed as a bore with a constant profiling.

6. The medical device according to claim 1, **characterised in that** the joint pin is designed with a centrally arranged guide section which has a larger cross-section than the clearance sections adjoining it on both sides, and **in that** the joint recess is designed as a stepped bore with a centrally arranged guide bore which has a smaller cross-section than the clearance bores adjoining it on both sides.

7. The medical device according to claim 1, **characterised in that** opposing surfaces (43, 44) of the joint limbs (15, 16) form a sliding guide with play with adjacent surfaces (45, 46) of the second joint part (18) and **in that** the joint pin (32; 52) and the joint recess (31; 51) are matched to one another in such manner that, when the second joint part (18) is tilted relative to the first joint part (17) about a tilting axis aligned transversely to the longitudinal axis (38; 55), there is an exclusively elastic deformation of the joint pin (32; 52).

8. The medical device according to one of the preceding claims, **characterised in that** an extension of the central contact region (33; 56) in the direction of the longitudinal axis (38; 55) is less than 30 percent, preferably less than 25 percent, of an extension of one of the clearance regions (36, 37; 57, 58) in the direction of the longitudinal axis (38; 55).

9. The medical device according to claim 2 or 3, **characterised in that** a cross-sectional area of the guide bore (56) is less than 50 percent, preferably less than 40 percent, in particular less than 30 percent, of a cross-sectional area of the clearance bore (57, 58).

10. The medical device according to claim 4 or 5, **characterised in that** a cross-sectional area of the clearance section (36, 37) is less than 50 percent, preferably less than 40 percent, in particular less than 30 percent, of a cross-sectional area of the guide section (33).

## Revendications

1. Dispositif médico-technique comportant un agencement d'articulation (11 ; 30 ; 50), qui est réalisé pour un mouvement de pivotement d'une première partie d'articulation (17) par rapport à une seconde partie d'articulation (18), comportant une première partie d'articulation (17), qui comprend deux branches d'articulation (15, 16) agencées à distance l'une de l'autre, ainsi qu'un boulon d'articulation (32 ; 52) s'étendant entre les branches d'articulation (15, 16) le long d'un axe longitudinal (38 ; 55), qui détermine un axe de pivotement (38 ; 55) de l'agencement d'articulation (11 ; 30 ; 50), ainsi que comportant une seconde partie d'articulation (18), qui est reçu par zones entre les deux branches d'articulation (15, 16) et qui est traversé par un évidement d'articulation (31 ; 51), dans lequel le boulon d'articulation (32 ; 52) est reçu de manière à pouvoir pivoter dans l'évidement d'articulation (31 ; 51), dans lequel le boulon d'articulation (32 ; 52) et l'évidement d'articulation (31 ; 51) présente une zone de contact centrale (33 ; 56) comportant un contact tactile entre le boulon d'articulation (32 ; 52) et l'évidement d'articulation (31 ; 51) et des zones de dégagement (36, 37 ; 57, 58) se raccordant des deux côtés à la zone de contact (33 ; 56), s'étendant jusqu'aux branches d'articulation (15, 16) au moins partiellement à l'intérieur de l'évidement d'articulation (31 ; 51) et sans contact tactile entre le boulon d'articulation (32 ; 52) et l'évidement d'articulation (31 ; 51), pour assurer une transmission de force exclusivement centrale entre le boulon d'articulation (32 ; 52) et l'évidement d'articulation (31 ; 51).

2. Dispositif médico-technique selon la revendication 1, **caractérisé en ce que** l'évidement d'articulation (51) est réalisé sous la forme d'un alésage étagé comportant un alésage de guidage (56) agencé au centre, qui présente une section transversale inférieure à celle des alésages de dégagement (57, 58) qui lui sont adjacents de part et d'autre.

3. Dispositif médico-technique selon la revendication 2, **caractérisé en ce que** l'e boulon d'articulation (52) est réalisé avec un profilage constant entre les branches d'articulation (15, 16).

4. Dispositif médico-technique selon la revendication 1, **caractérisé en ce que** le boulon d'articulation (32) est réalisé avec une section de guidage (33) agencée au centre, qui présente une section transversale supérieure à celle des sections de dégagement (36, 37) qui lui sont adjacentes de part et d'autre.

5. Dispositif médico-technique selon la revendication 4, **caractérisé en ce que** l'évidement d'articulation (31) est réalisé sous la forme d'un alésage avec un profilage constant.

6. Dispositif médico-technique selon la revendication 1, **caractérisé en ce que** le boulon d'articulation est réalisé avec une section de guidage agencée au centre, qui présente une section transversale supérieure à celle des sections de dégagement qui lui sont adjacentes de part et d'autre, et **en ce que** l'évidement d'articulation est réalisé sous la forme d'un alésage étagé comportant un alésage de guidage agencé au centre, qui présente une section inférieure à celle des alésages de dégagement qui lui sont adjacents de part et d'autre.

7. Dispositif médico-technique selon la revendication 1, **caractérisé en ce que** des surfaces (43, 44) opposées l'une à l'autre des branches d'articulation (15, 16) forment avec des surfaces (45, 46) voisines de la seconde partie d'articulation (18) un guidage coulissant avec jeu et **en ce que** le boulon d'articulation (32 ; 52) et l'évidement d'articulation (31 ; 51) sont adaptées l'un à l'autre de telle sorte que, lors d'un basculement de la seconde partie d'articulation (18) par rapport à la première partie d'articulation (17) autour d'un axe de basculement transversal à l'axe longitudinal (38 ; 55), une déformation exclusivement élastique du boulon d'articulation (32 ; 52) se produit.

8. Dispositif médico-technique selon l'une des revendications précédentes, **caractérisé en ce qu'**une extension de la zone de contact centrale (33 ; 56) dans la direction de l'axe longitudinal (38 ; 55) vaut moins de 30 pour cent, de préférence moins de 25 pour cent, d'une extension de l'une des zones de dégagement (36, 37 ; 57, 58) dans la direction de l'axe longitudinal (38 ; 55).

9. Dispositif médico-technique selon la revendication 2 ou 3, **caractérisé en ce qu'**une surface de section transversale de l'alésage de guidage (56) vaut moins de 50 pour cent, de préférence moins de 40 pour cent, en particulier moins de 30 pour cent, d'une surface de section transversale de l'alésage de dégagement (57, 58).

10. Dispositif médico-technique selon la revendication 4 ou 5, **caractérisé en ce qu'**une surface de section transversale de la section de dégagement (36, 37) vaut moins de 50 pour cent, de préférence moins de 40 pour cent, en particulier moins de 30 pour cent, d'une surface de section transversale de la section de guidage (33).
